Europäisches Patentamt

(19) European Patent Office

Office européen des brevets

(11) Publication number: **0 083 155**

**A1**

(12) **EUROPEAN PATENT APPLICATION**

(21) Application number: 82306199.9

(22) Date of filing: 22.11.82

(51) Int. Cl.³: **A 61 F 1/03**
     A 61 F 1/04, F 16 F 3/08

(30) Priority: 07.12.81 US 327857

(43) Date of publication of application:
     06.07.83 Bulletin 83/27

(84) Designated Contracting States:
     DE FR GB

(71) Applicant: DOW CORNING CORPORATION

Midland Michigan 48640(US)

(72) Inventor: Winfield, Daniel Ludlam
     3329 Shelby Street
     Bartlett Tennessee(US)

(72) Inventor: Lewis, Frank Michael
     80 V. King Drive Est
     Cordova Tennessee(US)

(72) Inventor: Pafford, John A.
     6045 Flynthill
     Bartlett Tennessee(US)

(74) Representative: Kyle, Diana et al,
     ELKINGTON AND FIFE High Holborn House 52/54 High
     Holborn
     London WCIV 6SH(GB)

(54) **Shock absorbing stop for prosthetic devices.**

(57) There is described a shock absorbing stop (17) for hinge type prosthetic devices comprising a first body (19) of relatively low modulus elastomeric material positioned to make first contact with a movable force transmitting element of the prosthesis, space (21) adjacent the first body to receive lateral elastic flow of the elastomeric material, and a second body (18) of relatively higher modulus material for making contact with the force transmitting element after the space has been filled by lateral flow of the lower modulus material. The bulk modulus of the lower modulus material and the elastic modulus of the higher modulus material then serve to support the static loading.

*Fig. 2*

EP 0 083 155 A1

# SHOCK ABSORBING STOP FOR PROSTHETIC DEVICES

The present invention relates in general to prosthetic joints of the semi-constrained type and more particularly to improvements in mechanical stops for inclusion in such devices.

In the field of implantable prosthetic joints, a common type of joint employs two prosthetic elements implanted in adjacent bones forming a skeletal joint. The movement of one prosthetic member with respect to the second member may be controlled by the curvature of the mating surfaces, or the prosthetic members may be connected by a hinge pin such that the two elements move with respect to one another around the hinge pin axis. Another similar type of prosthetic joint is provided with a rotational axis which is not rigidly fixed to allow for relatively minor movements in directions other than about the primary rotational axis. Such prostheses have found application as replacements for diseased or destroyed knee, elbow, and finger joints.

Joint prostheses of the semi-constrained type have proven useful in reconstruction of severely disabled and unstable joints in which the normal ligamentous support has been severely impaired. The prostheses therefore must supply stabilizing mechanisms which would normally be accomplished by the appropriate ligaments. In such cases, this is accomplished by designing into the prosthesis a limitation in the amount of relative rotational movement between the adjacent prosthetic joint elements. Typically the prosthesis includes a mechanical stop to prevent hyperextension of the reconstructed joint and some designs may also include a mechanical stop to limit the amount of flexion or axial rotation allowed by the prostheses.

Dadurian (U.S. Patent No. 4,112,522) and Laurie (U.S. Patent No. 4,134,158) have described hinge type prostheses for replacement of knee joints which employ mechanical stops to limit rotational motion. Frey (U.S. Patent No. 4,183,104) has described a prosthetic joint for reconstruction of the elbow which also includes a hyperextension stop. In these patents metal and/or rigid plastic materials are suggested as materials of construction. Typically the materials would be surgical alloys such as stainless steel or cobalt-chrome alloy, or a plastic material such as high density polyethylene or ultra high molecular weight polyethylene. These materials form relatively rigid mechanical stops, thus transmitting impact forces to implanted components of the prosthetic joint and ultimately to the prosthesis-bone interface. Aside from possibly causing clicking sounds which are disconcerting to the patient, such forces when transmitted to the prosthesis-bone interface can cause failure of the bone cement used to attach the prosthesis to the bone, or even resorption of the bone in the vicinity of the prosthesis. Such cement failure or bone resorption will eventually result in loosening and failure of the function of the prosthetic device.

Patrick and McDaniel (U.S. Patent No. 4,136,405) have attempted to eliminate this disadvantage by employing resilient material as a mechanical stop. In a commercial design having a mechanical stop similar to that used in their prosthesis, silicone elastomer is utilized. While short term application of this system is satisfactory, silicone elastomer seems incapable of supporting the extremely high forces inherent in the knee joint for the number of cycles that would be the normally expected useful life of the prosthesis. Failure and fragmentation of the silicone elastomer hyperextension stop have been reported to occur in time periods of from one to five years after implantation.

Accordingly, it is an object of the present invention to provide a new and improved mechanical stop for limitation of motion of semi-constrained type joint prostheses. More particularly, it is an object of the invention to provide a mechanical stop which will cushion and attenuate impact forces and be of sufficient mechanical strength to preclude failure even after extended use.

In accordance with these and other objects, there is provided by the present invention a mechanical stop for a semi-constrained type prosthetic device which stop mechanism is composed of at least two adjacent materials: one being a relatively low elastic modulus material having free space provided to accommodate lateral elastic flow under deformation while absorbing initial forces, and the other being a relatively higher modulus material positioned to carry a major portion of the load under static conditions, the combination being selected to support the maximum static load that will be applied. A suitable exemplary embodiment for a semi-constrained knee prosthesis mechanical stop is made up of a body of high strength silicone rubber surrounded by a body of ultra high molecular weight polyethylene. The mechanical stop is mounted in the tibial portion of the prosthesis and is designed so that the silicone rubber material first receives contact with the femoral condylar portion of the prosthesis as the leg is extended. Sufficient space is provided for elastic lateral flow of the silicone rubber to occur as the motion is decelerated by absorption of the forces. The amount of material and free space are chosen so that the silicone rubber material fills the free space and then simultaneously loading begins to occur on the high molecular weight polyethylene which due to its higher elastic modulus acts to withstand the major weight bearing forces under static loading conditions.

If desired, more than two materials can be used to act as a mechanical stop by choosing one or more materials intermediate in modulus such that the lowest modulus material is the first to absorb forces and attenuate motion, then the next lowest and so forth. As motion is arrested forces are taken up by materials of increasing modulus until the final static load is primarily supported in part by the highest modulus material.

The devices of the present invention act to gradually decelerate the prosthetic joint by use of the low modulus material but then offer high modulus material to accept heavy static loading and prevent overloading of the low modulus material. This arrangement effectively functions as a preventative against transmission of shock forces to the bone-joint interfaces, thereby reducing the tendency of the joint to loosen due to failure of cement or bone resorption. Additionally it is expected to lengthen the life of the stop member itself in comparison with prior art devices. Although the system was designed for use in implantable prosthetic joints the same principles are applicable to certain external prosthetic joints.

The invention will become better understood by those skilled in the art from a consideration of the following detailed description when read in connection with the accompanying drawings wherein:

Fig. 1 is a side elevational view of a prosthetic knee joint incorporating the mechanical stop of the present invention;

Fig. 2 is a fragmentary cross-sectional view of a portion of the joint of Fig. 1 illustrating details of the mechanical stop;

Fig. 3 is a fragmentary cross-sectional view similar to that of Fig. 2 but showing the low modulus material partially loaded by the femoral condyle of Figs. 1 and 2.

Fig. 4 is a fragmentary cross-sectional view similar to Figs. 2 and 3 but showing the joint in a position of full stop extension;

Fig. 5 is a fragmentary cross-sectional view similar to Fig. 2 showing a modified design of the joint and stop; and

Fig. 6 is a fragmentary cross-sectional view similar to that of Fig. 3 showing the modification of Fig. 5 in the full stop position.

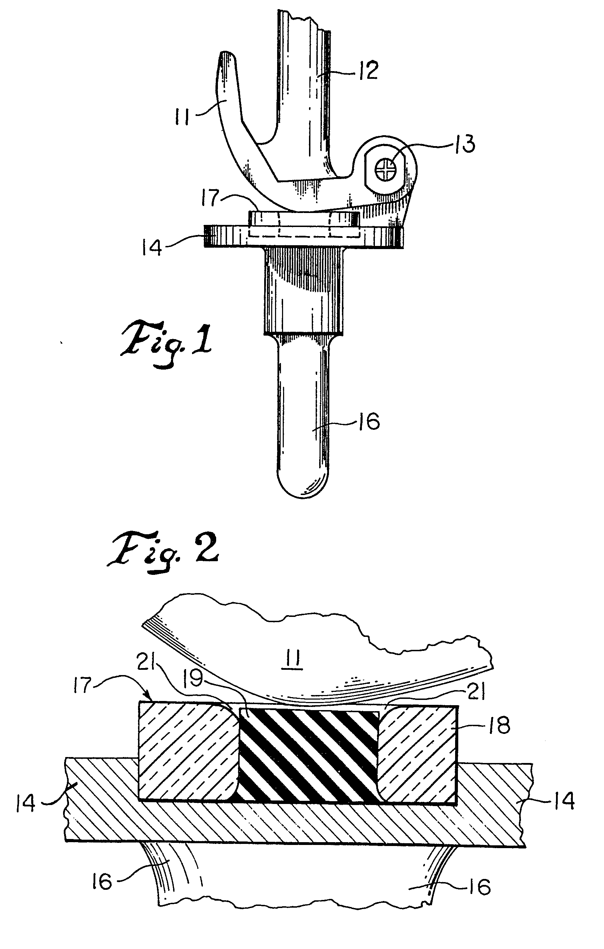

Referring now to the drawings wherein like reference characters designate like or corresponding parts throughout the figures thereof there is shown in Fig. 1 a hinged type implantable knee prosthesis having a femoral element comprising a femoral condylar portion 11 attached to a fixation stem 12 and hinged by means of a pin 13 to a tibial element comprising a tibial plateau component 14 affixed to a fixation stem 16. The condylar and tibial elements are typically made of surgically acceptable metals such as stainless steel or cobalt-chrome alloy. This type of prosthesis is implanted as a replacement for the human knee by suitable preparation of the distal end of the femur and proximal end of the tibia, applying cement, if desired, to prepared portions, inserting the fixation stems into the femur and tibia respectively and then inserting the pin 13 in the prosthesis to connect the two elements. In operation as the knee flexes and extends the tibial element rotates around the hinge pin 13 relative to the femoral element of the prosthesis. The condylar portions 11 approximate the shape of the normal femoral condyles of the human skeleton and allow cooperation with the patella. As the distal portion of the leg reaches the fully extended position the condylar portion of the prosthesis makes contact with the tibial plateau. The specific form of the prosthetic joint shown is for illustrative purposes and forms no part of the present invention. It is to be understood that the invention is applicable to various prosthesis designs.

In order to prevent sudden contact and resultant shock a stop member 17 made in accordance with the present invention is positioned in a recess in the tibial plateau in a position to accept contact with the femoral portion of the prosthesis as the leg is extended. The stop member 17 and its function can be seen more clearly from Figs. 2-4 which show sequential cross-sectional views of a stop member in operation.

As may be seen from these figures the stop member 17 comprises an outer body 18 of relatively high elastic modulus material such as polyethylene, polypropylene, polyacetal, polyester, or the like. In a preferred embodiment the body 18 is made of ultra high molecular weight polyethylene. An inner body or core body 19 of lower elastic modulus than that of the outer body 18 is located in an aperture in the outer body but does not completely fill the aperture as can be seen by the space 21 of Fig. 2. In a preferred embodiment high performance silicone elastomer is used to form the core body 19, although it is to be realized that other biocompatible materials which have a relatively low elastic modulus and sufficient strength and toughness can also be used.

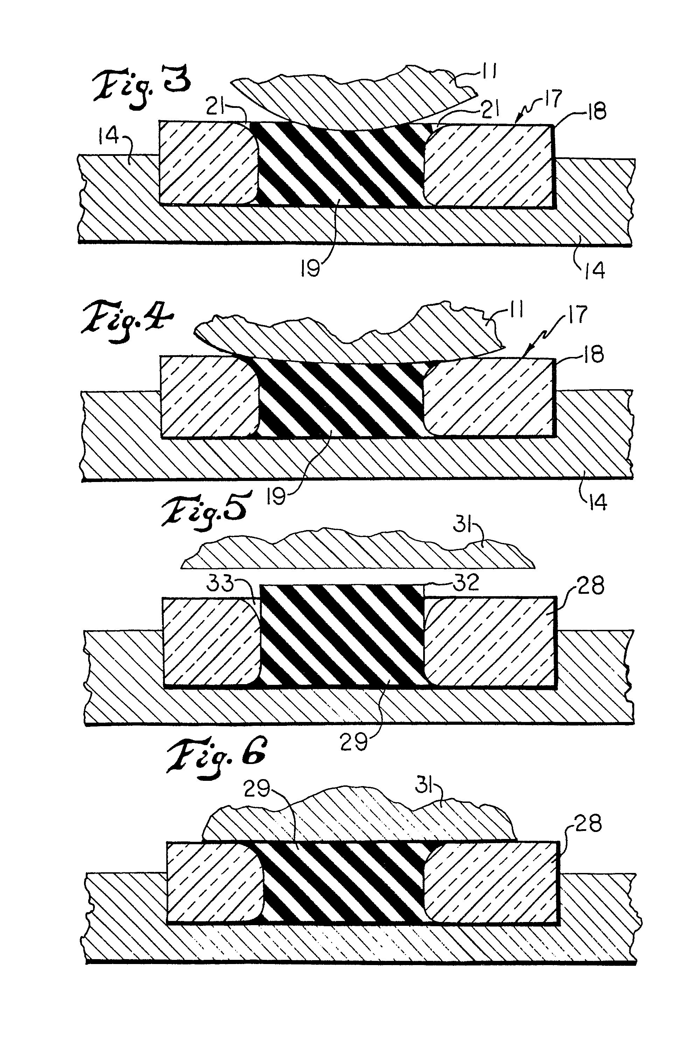

The operation of the device can be seen more clearly by viewing Figs. 2, 3 and 4 sequentially. In Fig. 2 the condylar portion 11 of the femoral prosthesis element is shown in the position in which it is first making contact with the core 19 as the leg is extending. As the leg extends further, as may be seen in Fig. 3, loading of the core 19 causes lateral elastic flow of the core material into the free space 21 and a cushioning effect is provided by the material as forces being applied are being resisted by the material. As load continues to be applied and the material flows due to the curvature of the condyles the effective modulus of the core material gradually increases. Once the space 21 is filled and the core material 19 becomes completely confined, contact is made between the condyle 11 and the outer portions 18 of the stop member as shown in Fig. 4. At this point, the bulk modulus of the confined material and the elastic modulus of the higher modulus material are both acting to resist further extension, the majority of the load being carried by the outer higher modulus material. In the preferred embodiment the amount of free space for elastic flow of the lower modulus material is chosen so that the space becomes filled simultaneously with the time that the higher modulus material becomes loaded by the condyles of the femoral portion of the knee prosthesis.

An analysis of the action of the stop shows that the apparent modulus of the structure varies with the position of the condyle as it loads the stop. The apparent modulus, $E_c$, can be determined by the equation:

$$\frac{1}{E_c} \qquad \frac{1}{E_e(1 + 2kS^2)} \qquad \frac{1}{E\infty} \qquad ,$$

where
$E_c$ = Compressive Modulus
$E_e$ = Elastic Modulus
$E\infty$ = Bulk Modulus
$k$ = Empirical constant
$S$ = Shape factor = $\dfrac{\text{loaded area}}{\text{force-free area}}$ .

As the low modulus material deforms and begins to fill the free space around it the force-free area of the material decreases in size relative to the loaded area of the material's surface thus increasing the shape factor. This results in an increase in the apparent compressive modulus. As an example of theoretical analysis of the device assume a shape factor of $S =$ 1.24, which would occur sometime after contact between the curved condyle and the low modulus body. With the high performance silicone elastomer $E_e \simeq 460$ pounds per square inch (psi), $E\infty \simeq 150,000$ psi, $k \simeq 0.64$ and $E_c \simeq 1365$ psi. As S continues to increase $E_c$ also continues to increase and the motion is attenuated at a greater rate. Once the free space around the low modulus material is completely eliminated, the apparent modulus of the low modulus material becomes the bulk modulus of the material. A smooth load transmission can be obtained by selection of design and materials such that the bulk modulus of the low modulus elastomeric material is of
approximately the same magnitude as the elastic modulus
of the high modulus plastic. By using high performance
silicone elastomer having a bulk modulus of $1.5 \times 10^5$
pounds per square inch and ultra high molecular weight
polyethylene with a compressive modulus of approximately
$1.3 \times 10^5$ pounds per square inch as loading of the high
modulus material begins a relatively smooth transition
is achieved.

As shown in Figs. 5 and 6 in the event a shock
absorbing stop is required to be used against a
substantially flat force transmitting surface 31 the
body 29 of low modulus material must initially extend
above the surface of the body of high modulus material
28 as shown at 32. As the surface of the force
transmitting body 31 approaches the stop it first makes
contact with the body of low modulus material causing
horizontal elastic flow into the free space 33 which
again causes increase in the shape factor, resultant
increase in effective modulus, and attenuation of the
forces until the free space is filled and the force
transmitting surface makes contact with the higher
modulus material at which point the load is carried by
both the high and low modulus bodies.

While the embodiments specifically described
show the use of two materials it is to be understood
that a third or more materials of intermediate modulus
can be interposed between the high and low modulus
bodies. The lowest modulus material is placed to be the
first to absorb forces. As motion is attenuated, the
forces are taken up sequentially by materials of
increasing modulus until the final static load is
supported in large part by the highest modulus material.

While the device has been described particularly with respect to implantable prostheses it should be understood that the same principles can be applied to external prosthetic devices, although the lack of need for biological compatibility in such instances allows for greater choice of suitable materials.

It should also be understood by those skilled in the art that the stop mechanism of this invention can be utilized in connection with any joint prosthesis in which a pair of elements are made moveable relative to one another and relative movement between the elements must be arrested. The unit is placed in a position where the lowest modulus material makes first contact between the elements and the highest modulus material is located to make contact between the elements after substantial attenuation of their motion relative to one another.

Obviously other modifications and variations of the embodiments of a shock absorbing stop for prosthetic devices which have been described herein will become obvious to those skilled in the art from a consideration of the foregoing. Therefore it is to be understood that within the scope of the appended claims the invention can be practiced otherwise than as specifically described.

CLAIMS

1.    A stop mechanism for arresting movement relative
to one another of a pair of elements of a prosthetic joint
characterised in that the stop mechanism (17) comprises
          a first body (19) of relatively low elastic modulus
elastomeric material positioned in a location to
make first contact between the elements and absorb forces
transmitted thereby and ettenuate relative motion thereof,
          a second body (18) of higher elastic modulus material
than that of the first body positioned adjacent the first
body in a location to make contact between the elements after
substantial attenuation of the relative motion thereof
by the first element, and
          a volume of free space (21) adjacent the first
body for receiving lateral elastic flow of the material
of the first body as forces are applied to the first body
by relative movement of the elements; the volume of free
space being that required wholly to confine the first body
as contact between the second body and the elements occurs,
          whereby static loading between the elements is
supported by both the first and second bodies.
2.    A prosthetic joint having a pair of elements
movable relative to one another including a stop
mechanism for arresting the movement as the
elements approach one another characterised in that the stop
mechanism (17) comprises:
          a first body (19) of relatively low elastic
modulus elastomeric material positioned in a location to
make first contact between the elements and absorb forces
trasmitted thereby and attenuate relative motion thereof,
          a second body (18) of higher elastic modulus
material than that of the first body positioned adjacent
the first body in a location to make contact between the
elements after substantial attenuation of the relative motion thereof by the first element, and

a volume of free space (21) adjacent the first body for receiving lateral elastic flow of the material of the first body as forces are applied to the first body by relative movement of the elements; the volume of free space being that required wholly to confine the first body as contact between the second body and the elements occurs,

whereby static loading between the elements is suppported by both the first and second bodies.

3. A device according to claim 1 or 2, wherein said prosthetic joint is a knee prosthesis and said stop mechanism is mounted in a recess in the tibial portion of said prosthesis for coaction with the femoral portion.

4. A device according to any of claims 1 to 3, and further including a third body of material having an elastic modulus which is intermediate the materials of the first and second bodies, the third body being positioned to make initial contact between the elements after the first body but before the second body.

5. A device according to any of claims 1 to 4, wherein the magnitudes of the bulk modulus of the first material and the elastic modulus of the second material are approximately the same.

6. A device according to any of claims 1 to 5 wherein the prosthetic joint is a surgically implantable knee prosthesis, the stop mechanism is mounted in a recess in the tibial portion of the prosthesis for coaction with the femoral portion, the first material is a silicone elastomer and the second material is a high molecular weight polyethylene.

*Fig. 1*

*Fig. 2*

**Fig. 3**

**Fig. 4**

**Fig. 5**

**Fig. 6**

0083155

## European Patent Office

## EUROPEAN SEARCH REPORT

Application number

EP 82 30 6199

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y,A | DE-A-2 227 090  (GEBR. SULZER AG)<br>* Claim 3 ; figure 5 * | 1,2,3, 6 | A 61 F   1/03<br>A 61 F   1/04<br>F 16 F   3/08 |
| Y | DE-A-2 310 343  (INTERNATIONAL HARVESTER CO.)<br>* Claims 1, 2 * | 1,2 | |
| Y | US-A-2 183 076  (KAISER)<br>* Figure 1 * | 1,2 | |
| Y | WO-A-8 100 606  (CATERPILLAR TRACTOR CO.)<br>* Claims 1, 3-5 * | 1,2 | |
| A | GB-A-1 475 688  (SILENTBLOC LTD.) | | TECHNICAL FIELDS SEARCHED (Int. Cl. 3) |
| A | US-A-1 504 903  (ROWLEY) | | A 61 F   1/00<br>F 16 F   3/00 |
| A | Patent Abstracts of Japan, vol. 1, no. 101, 8 September 1977, page 2903M77 & JP-A-52-41775 | | |
| D,A | US-A-4 112 522  (DADURIAN et al.) | | |
| D,A | US-A-4 134 158  (LAURE) | | |
| | ---        -/- | | |

The present search report has been drawn up for all claims

| Place of search<br>BERLIN | Date of completion of the search<br>25-02-1983 | Examiner<br>KANAL P K |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82